# Europäisches Patentamt

## European Patent Office

## Office européen des brevets

(19)

(11) Veröffentlichungsnummer: **0 106 957**
**B1**

(12) **EUROPÄISCHE PATENTSCHRIFT**

(45) Veröffentlichungstag der Patentschrift:
**18.03.87**

(21) Anmeldenummer: **83107858.9**

(22) Anmeldetag. **09.08.83**

(51) Int. Cl.⁴: **C 07 D 493/04,** C 07 D 307/20 //
(C07D493/04, 307:00, 307:00)

(54) Verfahren zur Gewinnung reiner kristalliner Anhydropentite, Mono- und/oder Dianhydrohexite.

(30) Priorität: **14.08.82 DE 3230349**

(43) Veröffentlichungstag der Anmeldung:
**02.05.84 Patentblatt 84/18**

(45) Bekanntmachung des Hinweises auf die Patenterteilung:
**18.03.87 Patentblatt 87/12**

(84) Benannte Vertragsstaaten:
**BE CH DE FR GB IT LI NL SE**

(56) Entgegenhaltungen:
**EP-A-0 052 295**
**US-A-2 390 395**
**US-A-3 160 641**
**US-A-3 484 459**

(73) Patentinhaber: **CPC INTERNATIONAL INC.,
International Plaza P.O. Box 8000, Englewood
Cliffs New Jersey 07632 (US)**

(84) Benannte Vertragsstaaten:
**BE CH DE FR GB IT LI NL SE**

(73) Patentinhaber: **Maizena Gesellschaft mbH,
Postfach 10 43 20 Spaldingstrasse 218, D-2000
Hamburg 1 (DE)**

(84) Benannte Vertragsstaaten: **DE**

(72) Erfinder: **Feldmann, John, Grenzstrasse 151, D-4150
Krefeld (DE)**
Erfinder: **Koebernik, Hubert, Hohenzollernstrasse
76, D-4150 Krefeld (DE)**
Erfinder: **Woelk, Hans- Ulrich, Friedrich-
Kristenstrasse 3, D-2000 Hamburg 65 (DE)**
Erfinder: **Richter, Klaus, Geisweg 3A, D-4005
Meerbusch Bösinghoven (DE)**

(74) Vertreter: **Patentanwälte Müller- Boré, Deufel,
Schön, Hertel, Lewald, Otto, Postfach 26 02 47
Isartorplatz 6, D-8000 München 26 (DE)**

**0 106 957**

**Beschreibung**

Die Erfindung betrifff ein Verfahren zur Gewinnung reiner kristalliner Anhydropentite Mono- und/oder Dianhydrohexite ($C_{5/6}$-Anhydrozuckeralkohole) durch Kristallisation aus einer Lösung.

$C_{5/6}$-Anhydrozuckeralkohole sind seit langem bekannte Verbindungen, die in der Regel durch säurekatalysierte Wasserabspaltung bei erhöhter Temperatur und in flüssigen Reaktionssystemen aus den entsprechenden Zuckeralkoholen hergestellt werden und - an sich - vielfältige Verwendung, vor allem als Zwischenprodukte, insbesondere als Polyolkomponente für Polyetter- und Polyurethankunststoffe, finden können, bislang aber vor allem deswegen doch keine erhebliche praktische Bedeufugg erlangt haben, weil ihre Abtrennung aus den bei der Herstellung anfallenden, mehr oder weniger komplexen Reaktionsgemischen in der für bestimmte Zwecke, insbesondere die Polyurethanherstellung, erforderlichen Reinheit dach dem Stand der Technik nicht oder nur mit untragbar hohem Aufwand gelingt.

Die rohen Reaktionsproduktgemische enthalten als Hauptbesfandteile $C_{5/6}$-Anhydrozuckeralkohol(e), nicht umgesetztes Ausgaggsmaterial, sowie gegebenenfalls Lösungsmittel und - bei der tislang immer noch gebräuchlichsten Verwendung homogener, d. h. molekulardispers im Reaktionsgemisch verteilfer Säurekatalysatoren - gelösten Katalysator sowie geringe, aber gleichwohl für verschiedene Anwendungszwecke störende Mengen an Estern der Katalysatorsäure und verschiedenen Neben-, Abbau- und Folgeprodukten.

Letztere werden vor der eigentlichen Aufbereitung des Reaktionsproduktgemisches meist nach in der Zuckerchemie üblichen Methoden durch Behandlung mit Ionenaustauscherharzen und/oder Aktivkohle entfernt.

Die Gewinnung des gewünschten $C_{5/6}$-Anhydrozuckeralkohols aus dem so vorgereinigten Reaktionsgemisch erfolgt dann nach durchweg mehrstufigen Verfahren, wobei in der Regel verschiedene Trennmethoden in Kombination angewandt werden.

Dabei führt man meist zunächst eine Vortrennung durch Chromatographie. Extraktion und/oder insbesondere fraktionierende Destillation unter vermindertem Druck durch, worauf das gewonnene $C_{5/6}$-Anhydrozuckeralkoholrohprodukt durch - in der Regel mehrmalige - Kristallisation aus Lösungsmitteln, insbesondere Essigester, gegebenenfalls ein Gemisch mit niedrigen aliphatischen Alkoholen, gereinigt wird.

Da selbst diese aufwendige Trennung und Reinigung häufig kein Produkt mit einer für die bestimmungsgemässe Verwendung erforderlichen Reinheit liefert und/oder die Ausbeuten sehr gering sind, wendet man häurig zusätzlich chemische Hilfsmittel, wie Borationen an, um schwer entfernbare Verunreinigungen zu binden (US-PS 3. 160. 641) oder setzt das Rohproduktgemisch, z. B. durch Acetalisieren, in besser trennbare Derivate um, aus denen der gewünschte $C_{5/6}$-Anhydrozuckeralkohol nach der Trennung wieder freigesetzt wird (US-PS 3. 484.459).

Trotz dieser extrem aufwendigen und unwirtschaftlichen Trennverfahren enthalten die gewonnenen kristallinen $C_{5/6}$-Anhydrozuckeralkohole meist noch Spuren von Verunreinigungen, insbesondere Lösungsmittelreste wie Essigester, die bei bestimmten Verwendungszwecken, vor allem der Herstellung von Polyestern erheblich stören.

Der Erfindung lag daher die Aufgabe zugrunde ein Verfahren zur Gewinnung reiner kristalliner $C_{5/6}$-Anhydrozuckeralkohole zu schaffen, das die Nachteile des Standes der Technik überwindet und es insbesondere ermöglicht, die $C_{5/6}$-Anhydrozuckeralkohole durch einfache Kristallisstion ohne organische Lösungsmittel aus lediglich gegebenenfalls in üblicher Weise mit Ionenaustauschern und/oder Aktivkohle gereinigten und/oder aufkonzentrierten Reaktionsproduktgemischen wirtschaftlich und in hoher Ausbeute und Reinheit zu gewinnen.

Diese Aufgabe wird erfindungsgemäss in der im Hauptanspruch gekennzeichneten Weise ausgehend von der Erkenntnis gelöst, dass sich $C_{5/6}$-Anhydrozuckeralkohole trotz ihrer sogar im Vergleich zu den entsprechenden Zuckeralkoholen und Zuckern extrem hohen Wasserlöslichkeit aus wässrigen Lösungen bzw. Schmelzen überraschenderweise mit vergleichsweise hoher Selektivität und Reinheit nach für die Kristallisation von Zuckern und einigen Zuckeralkoholen an sich bekannten Methoden kristallisieren lassen.

Die Erfindung betrifft demgemäß ein Verfahren zur Gewinnung reiner kristalliner Anhydropentite, Mono- oder Dianhydrohexite ($C_{5/6}$-Anhydrozuckeralkohole) durch Kristallisation aus einer Lösung und ist dadurch gekennzeichnet, daß man

eine wäßrige Lösung mit einem Trockensubstanzgehalt von mindestens 70 Gew.-%, die, bezogen auf Trockensubstanz, mindestens 40 Gew.-% des zu kristallisierenden $C_{5/6}$-Anhydrozuckeralkohols und gegebenenfalls als Rest einen oder mehrere andere(n) $C_{5/6}$-Zuckeralkohol(e), $C_{5/6}$-Anhydrozuckeralkohol(e) und/oder herstellungsbedingte Verunreinigungen enthält (Dicksaft),

mit mindestens 0,1 Gew.-% Impfkristallen des zu gewinnenden $C_{5/6}$-Anhydrozuckeralkohols animpft,

einen erheblichen Teil des in der eingesetzten Lösung enthaltenen zu gewinnenden $C_{5/6}$-Anhudrozuckeralkohols bei einer Temperatur von höchstens 70°C auskristallisieren läßt, wobei man die Übersättigung der Lösung (Mutterlauge) in Bezug auf den zu kristallisierenden $C_{5/6}$-Anhydrozuckeralkohol durch

Verdampfen von Wasser, Zufuhr von Dicksaft und/oder langsame Temperatursenkung mit einer Geschwindigkeit von höchstens 0,1°C/Minute

in einem Bereich hält, in dem die vorhandenen Kristalle weiterwachsen, jedoch praktisch keine Spontankristallkeimbildung erfolgt,

2

0 106 957

und

den auskristallisierten $C_{5/6}$-Anhydrozuckeralkohol von der Mutterlauge abtrennt und

die abgetrennten Kristalle gegebenenfalls mit möglichst wenig Wasser oder einer wäßrigen Lösung des betreffenden $C_{5/6}$-Anhydrozuckeralkohols wäscht und/oder trocknet

Vorzugsweise verfährt man so, da. die von den $C_{5/6}$-Anhydro-zuckeralkoholkristallen abgetrennte Mutterlauge, gegebenenfalls nach Einengung, zumindest teilweise

einer Umsetzung von $C_{5/6}$-Zuckeralkohol(en) in $C_{5/6}$-Anhydrozuckeralkohol(e) und/oder von Mono- in Dianhydrohexit(e) durch säurekatalysierte Wasserabspaltung unterworfen und/oder

als Dicksaft mindestens einer weiteren Kristallisation zu- und/oder zurückgeführt wird, in der der gleiche wie oder ein anderer $C_{5/6}$-Anhydrozuckeralkohol als der bei der ersten bzw. der vorhergegangenen Kristallisation gewonnene auskristallisiert wird.

Gemäß einer weiteren bevorzugten Ausführungsform wird in der Weise vorgegangen, daß von einem Dicksaft ausgegangen wird, der nach der Abtrennung eines ersten kristallinen $C_{5/6}$-Anhydrozuckeralkohols eine Mutterlauge liefert, die, bezogen auf Trockensubstanz, mindestens 40 Gew.-% mindestens eines zweiten $C_{5/6}$-Anhydrozuckeralkohols und/oder durch säurekatalysierte Wasserabspaltung in den zweiten $C_{5/6}$-Anhydrozuckeralkohol überführbare(r) Verbindung(en) enthält, und daß man die gegebenenfalls aufkonzentrierte und/oder einer säurekatalysierten Wasserabspaltung unterworfene Mutterlauge aus der ersten Kristallisation mit mindestens 0,1 Gew.-% kristallinem zweitem $C_{5/6}$-Anhydrozuckeralkohol animpft.

Wie bereits erwähnt gelingt die Kristallisation - unter ansonsten günstigen Bedingungen - sogar bereits aus Lösungen mit nur etwa 70 % Trockensubstanzgehalt, jedoch arbeitet man zweckmässigerweise mit höher konzentrierten Lösungen bzw. Schmelzen mit einem Wassergehalt von höchstens 20, vorzugsweise höchstens 15 und insbesondere nicht mehr als 8 Gewichtsprozent.

Dabei sollte jedoch ein gewisser Mindestgehalt an Wasser nicht unterschritten werden, und es empfiehlt sich, Dicksäfte mit einem Wassergehalt von mindestens 1 und zweckmässigerweise mindestens 2 Gew. -% einzusetzen. Optimale Ergebnisse erzielt man erfahrungsgemäss mit Dicksäften, deren Wassergehalt bei etwa 3 Gew. -% liegt.

Der Gehalt des Dicksaftes an dem jeweils zu kristallisierenden $C_{5/6}$-Anhydrozuckeralkohol kann - verglichen mit dem Stand der Technik ausserordentlich niedrig liegen und braucht im Extremfall, bezogen auf Trockensubstanz, nur etwa 40 Gew. -% zu betragen, so dass es nach dem Verfahren der Erfindung möglich ist, aaus mehrere $C_{5/6}$-Anhydrozuckeralkohole enthaltenden Dicksäften zwei oder sogar drei verschiedene $C_{5/6}$-Anhydrozuckeralkohole durch fraktionierende Kristallisation nacheinander in reiner kristalliner Form zu gewinnen, indem man den Dicksaft zunächst mit Kristallen eines $C_{5/6}$-Anhydrozuckeralkohols animpft und diesen so lange auskristallisieren lässt, bis sich ein weiterer $C_{5/6}$-Anhydrozuckeralkohol in der Mutterlauge auf über 40 Gew. -%. bezogen auf Trockensubstanz, angereichert hat, die Mutterlauge von den Kristallen des ersten $C_{5/6}$-Anhydrozuckeralkohols abtrennt, gegebenenfalls einengt und/oder einer säurekatalysierten Wasserabspalt ung zur Umsetzung von $C_{5/6}$-Zuckeralkohol(en) in $C_{5/6}$-Anhydrozuckeralkohol(e) und/oder von $C_6$-Mono- in $C_6$-Dianhydrozuckeralkohol(e) unterwirft und dann, gegebenenfalls nach üblicher Vorreinigung, mit Kristallen des angereicherten zweiten $C_{5/6}$-Anhydrozuckeralkohols animpft.

In diesem Zusammenhang ist darauf hinzuweisen, dass mit Hilfe des Verfahrens der Erfindung zwar bereits bei Einsatz von Dicksäften mit einen Gehalt von nur 40% an zu kristallisierendem $C_{5/6}$-Anhydrozuckeralkohol befriedigende Kristallisationsausbeuten und Produktreinheitsgrade erzielt werden können, reinere Dicksäfte in der Regel aber doch günstiger sind, und es sich daher empfiehlt, für das Verfahren Dicksäfte mit einem auf Trockensubstanz bezogenen Gehalt an zu kristallisierendem $C_{5/6}$-Anhydrozuckeralkohol von mindestens 50, vorzugsweise mehr als 60 und insbesondere mindestens 75 Gew. -% zu verwenden. Selbst der letztgenannte Wert liegt erheblich unter der nach dem Stand der Technik für erforderlich gehaltenen Reinheit.

Nach oben sind der Reinheit des Dicksaftes zwar keine technischen Grenzen gesetzt, jedoch verwendet man aus wirtschaftlichen Gründen bevorzugt Dicksäfte, die bezogen auf Trockensubstanz, nur bis zu 99, vorzugsweise bis zu 95 und insbesondere bis zu 90 Gew. -% des jeweils zu kristallisierenden $C_{5/6}$-Anhydrozuckeralkohols enthalt en.

Die Impfkristallmenge kann, wie weiter oben ausgeführt, beim Verfahren der Erfindung erstaunlich niedrig gehalten werden und braucht unter ansonsten günstigen Umständen fallweise nur etwa 0,1 Gew. -% zu betragen. jedoch sind - technisch - höhere Impfkristallmengen natürlich zweckmässig. Erfahrungsgemäss empfiehlt es sich beim Verfahren der Erfindung, den Dicksaft mit 0.2 bis 20, vorzugsweise 0,5 bis 10 und insbesondere etwa 1 bis 5 Gew. -% Kristallen des jeweils zu gewinnenden $C_{5/6}$-Anhydrozuckeralkohols anzuimpfen, wobei der jeweils optimale Kompromiss von den Umständen des Einzelfalls, insbesondere dem jeweils zu gewinnenden $C_{5/6}$-Anhydrozuckeralkohol, der Zusammensetzung des Dicksafts und den geforderten Produkteigenschaften abhängt.

Die Kristallisationstemperatur liegt aufgrund der hohen Löslichkeit der $C_{5/6}$-Anhydrozuckeralkohole relativ niedrig und sollte, wie bereits erwähnt, auch in der Anfangsphase 70°C nicht oder allenfalls geringfügig überschreiten. Andererseits führen auch extrem niedrige Temperaturen selbst in der Endphase der Kristallisation zu Schwierigkeiten, so dass es erfahrungsgemäss in der Regel zweckmässig ist, die Kristallisation bei Temperaturen im Bereich von etwa 20 bis 65, vorzugsweise 25 bis 60 und insbesondere 30 bis 55°C durchzuführen.

Dabei kann grundsätzlich sowohl isotherm als auch mit im Verlauf der Kristallisation absinkenden Temperaturen gearbeitet werden, wobei es von einer Reihe von Umständen abhängt, welche dieser

3

Alternativen im Einzelfall den Vurzug verdient, da sie tendenziell unterschiedliche Vor- und Nachteile aufweisen; während bei isothermer Kristallisation die Verfahrensführung meist schon deswegen einfacher, sicherer und genauer ist, weil eine jedenfalls im grosstechnischen Masstab nur schwer mit der erforderlichen Genauigkeit und Schnelligkeit regelbare Variable konstant gehalten wird, lassen sich beim Arbeiten mit abnehmenden Kristallisationstemperaturen, jedenfalls bei einstufiger Arbeitsweise, in der Regel bessere Kristallausbeuten erzielen. Häufig empfiehlt sich daher, eine Kombination anzuwenden, bei der zunächst isotherm kristallisiert und dann zur Ausbeuteerhöhung in der Endphase die Temperatur allmählich abgesenkt wird. Soweit man mit sinkenden Temperaturen arbeitet, sollte der Temperaturgradient in einem Bereich von etwa 0,01 bis 0,05° C pro Minute gehalten werden.

Hinzuweisen ist in diesem Zusammenhang noch darauf, dass es beim Verfahren der Erfindung zwar in der Regel, und zwar insbesondere dann, wenn in einem Durchgang relativ hohe Kristallisationsausbeuten angestrebt, Dicksäfte mit verhältnismässig geringer Reinheit eingesetzt und/oder geringe Impfkristallmengen angewandt werden, vorzuziehen, aber durchaus nicht in jedem Fall notwendig ist die Kristallisation bei insbesondere in der Anfangsphase niedrigen und möglichst konstanten Übersättigungsgraden durchzuführen. Anders gesagt, ist es beim Verfahren der Erfindung überraschenderweise auch möglich. den Dicksaft vor dem Animpfen durch Einengen und/oder Abkühlen stark zu übersättigen und die Übersättigung sich im Laufe der Kristallisation abbauen zu lassen. Diese Arbeitsweise ergibt zwar keine optimalen Ausbeuten pro Durchgang, ist aber fallweise deswegen vorzuziehen, weil dabei erfahrungsgemäss vergleichsweise hohe Kristallisationsgeschwindigkeiten erzielt werden, wie Beispiel 3 belegt.

Als Dicksaft eignen sich zwar im Prinzip alle wässrigen $C_{5/6}$-Anhydrozuckeralkohollösungen, die der im Hauptanspruch gegebenen Definition entsprechen unabhängig von ihrer Vorgeschichte, jedoch werden für diesen Zweck bevorzugt durch säurekatalysierte Wasserabspaltung aus Pentit(en), Hexit(en) und/oder Monoanhydrohexit(en), insbesondere unter Verwendung heterogener saurer Katalysatoren, vor alleni nach einem der in den DE-Offenlegungsschriften P 30 41 626 und P 30 41 673 beschriebenen Verfahren erhaltene Reaktionsproduktgemische verwendet, die sich direkt oder nach keinen grossen Aufwand erfordernden Reinigung, Entfärbung und/oder Einstellung auf einen Wassergehalt von höchstens 30 Gew. -% nach in der Zuckertechnologie an sich bekannten und üblichen Methoden einsetzen lassen.

Das Verfahren der Erfindung kann diskontinuierlich oder kontinuierlich und ein- oder mehrstufig durchgeführt werden, wobei im letztgenannten Fall die verschiedenen Stufen räumlich getrennt in jeweils eigenen Kristallisatoren und/oder nacheinander in einem oder mehreren getrennten Kristallisator(en) durchgeführt werden können.

Die Beispiele erläutern die Erfindung und deren Vorteile gegenüber dem Stand der Technik.

Als Dicksaft wurde jeweils eine durch säurekatalysierte Wasserabspaltung unter Verwendung eines stark sauren Kationenaustauscherharzes in der II+-Form (makroporöses Polystyrolsulfonsäureharz, 14% DVBvernetzt) aus dem (den) entsprechenden Zuckeralkohol(en) hergestelltes Reaktionsproduktgemiseh verwendet, das durch Behandlung mit Ionenaustauschern in der H+ und OH--Form und/oder Mischbettionentauschern sowie Aktivkohle (pulvrig oder granulär) vorgereinigt und entfärbt, sowie jeweils durch Vakuumverdampfung auf den in den Beispielen angegebenen Wassergehalt eingestellt wurde.

## Beispiel 1

Gewinnung von kristallisiertem 1,4; 3,6-Dianhydro-D-sorbit. Als Kristallisator wurde ein mit einem langsam drehenden Rührwerk und Doppelmantel zum Beheizen und Kühlen versehener Kessel verwendet.

Im Kristallisator wurden 3.200 g eines Anhydropolyolgemisches. das 3 Gew. -% Wasser und, bezogen auf Trockensubstanz. 82 Gew. -% Dianhydrosorbit enthielt, mit einer Temperatur von 53° C vorgelegt und auf 45° C abgekühlt. Dann wurde der Dicksaft unter ständigem Rühren mit 10 g 1,4; 3,6-Dianhydrosorbit (Körnung ca. 0,3 - 0,9 mm) angeimpft, worauf das Gemisch im Laufe von 16 Stunden unter langsamem Rühren allmänlich auf 35° C abgekühlt wurde.

Dann wurden die Kristalle durch Zentrifugieren von der Mutterlauge getrennt und danach mit wenig kaltem Wasser gewaschen (ca. 50 ml).

Das so erhaltene Kristallisationsprodukt (Ausbeute: 1. 270 g. entsprechend 49,9% der Theorie, d. h. bezogen auf 1,4; 3,6-Dianhydro-D-sorbit) wies folgende Kenndaten auf:

| | |
|---|---|
| Reinheit (HPLC) | grösser 99% |
| Schmelzpunkt | 62, 5° C |
| Drehwert | $[\alpha]_D^{20} = +45°$ (c = 0,8; $H_2O$) |
| Wassergehalt | kleiner 0,5% |

## Beispiel 2

Beispiel 1 wurde wiederholt, wobei abweichend davon 100 g Dicksaft mit einem Dianhydrosorbitgehalt von 84 Gew. -%, bezogen auf Trockensubstanz vorgelegt und 5 g Impfkristalle zugesetzt, sowie in 8 Stunden von 45

4

auf 35° C abgekühlt wurde(n).

Ausbeute:

41,2 g (49,1% der Theorie) 1,4;3,6-Dianhydro-D-sorbit mit folgenden Kenndaten:

| | |
|---|---|
| Reinheit (HPLC) | größer 99% |
| Schmelzpunkt | 60,8° C |
| Drehwert | $[\alpha]_D^{20} = + 44,3°$ (c = 0,8; $H_2O$) |
| Wassergehalt | kleiner 0,5 % |

**Beispiel 3**

Beispiel 1 wurde wiederholt, wobei abweichend davon 158 g Dicksaft mit einem Dianhydrosorbitgehalt von 84 Gew.-%. bezogen auf Trockensubstanz vorgelegt und 1 g Impfkristalle zugesetzt, sowie 4 Stunden bei 33° C gerührt wurde.

Ausbeute:

(30,9 % der Theorie)

39 g 1,4; 3,6-Dianhydro-D-sorbit mit folgenden Kenndaten:

| | |
|---|---|
| Reinheit (HPLC) | größer 99 % |
| Schmelzpunkt | 61° C |
| Drehwert | $[\alpha]_D^{20} = + 44,8°$ (c = 0,8; $H_2O$) |
| Wassergehalt kleiner | 0,5 % |

**Beispiel 4**

Beispiel 1 wurde wiederholt, wobei abweichend davon 14 429 g Dicksaft mit einem Dianhydrosorbitgehalt von 75 Gew.-%. bezogen auf Trockensubstanz vorgelegt und 100 g Impfkristalle zugesetzt, sowie in 24 Stunden von 45° C auf 30° C abgekühlt wurde.

Ausbeute: 4 200 g (40,4 % der Theorie) 1,4; 3,6 Dianhydro-D-sorbit mit folgenden Kenndaten:

| | |
|---|---|
| Reinheit (HPLC) | größer 99 % |
| Schmelzpunkt | 60,7° C |
| Drehwert | $[\alpha]_D^{20} = + 44,4°$ (c = 0,8; $H_2O$) |
| Wassergehalt kleiner | 0,5% |

**Beispiel 5**

Beispiel 1 wurde wiederholt, wobei abweichend davon 200 g Anhydropolyolgemisch mit 10 Gew. -% Wasser und einem Dianhydromannitgehalt von 96%, bezogen auf Trockensubstanz, mit einer Temperatur von 60 C vorgelegt und 0,5 g 1,4; 3,6-Dianhydro-D-mannit-Impfkristalle zugesetzt, sowie in 12 Stunden von 60° C auf 40° C abgekühlt wurde(n).

Ausbeute:

72 g (41,6 % der Theorie) 1,4; 3,6 Dianhydro-D-mannit mit folgenden Kenndaten:

| | |
|---|---|
| Reinheit (HPLC) | größer 99 % |
| Schmelzpunkt | 86,5° C |
| Drehwert | $[\alpha]_D^{20} = + 92,2°$ (c = 1; $H_2O$) |
| Wassergehalt | kleiner = 0,5 % |

**Beispiel 6**

Beispiel 5 wurde wiederholt, wobei abweichend davon 100 g Anhydropolyolgemisch mit 5 Gew.-% Wasser und einem Dianhydromannitgehalt von 75 % bezogen auf Trockensubstanz vorgelegt und 3 g Impfkristalle zugesetzt, sowie in 12 Stunden von 42° C auf 30° C abgekühlt wurde.

Ausbeute:

43 g (60 % der Theorie) 1,4; 3,6-Dianhydro-D-mannit mit folgenden Kenndaten:

| | |
|---|---|
| Reinheit (HPLC) | gräßer 99% |
| Schmelzpunkt | 86° C |
| Drehwert | $[\alpha]_D^{20} = + 92°$ (c = 1, $H_2O$) |
| Wassergehalt | kleiner 0,5 % |

**Beispiel 7**

Beispiel 1 wurde wiederholt, wobei abweichend 100 g Dicksaft mit 7,5 Gew. -% Wasser und einem Gehalt an 1,4 - Monoanhydro-D-sorbit von 66%, bezogen auf Trockensubstanz, mit einer Temperatur von 55°C vorgelegt und 1 g 1,4 Anhydro-D-sorbit-Impfkristalle zugesetzt, sowie in 18 Stunden von 55°C auf 30°C abgekühlt wurde.

Ausbeute:

36 g (58,9 % der Theorie) 1,4-Monoanhydro-D-sorbit mit folgenden Kenndaten:

| | |
|---|---|
| Reinheit (HPLC) | größer 99 % |
| Schmelzpunkt | 115°C |
| Drehwert | $[\alpha]_D^{20} = -22°$ (c = 1; $H_2O$) |
| Wassergehalt kleiner | 0,5 % |

**Patentansprüche**

1. Verfahren zur Gewinnung reiner kristalliner Anhydropentite, Mono- und/oder Dianhydrohexite ($C_{5/6}$-Anhydrozuckeralkohole) durch Kristallisation aus einer Lösung, dadurch gekennzeichnet, daß man

eine wäßrige Lösung mit einem Trockensubstanzgehalt von mindestens 70 Gew.-%, die, bezogen auf Trockensubstanz, mindestens 40 Gew.-% des zu kristallisierenden $C_{5/6}$-Anhydrozuckeralkohols und gegebenenfalls als Rest einen oder mehrere andere(n) $C_{5/6}$-Zuckeralkohol(e), $C_{5/6}$-Anhydrozuckeralkohol(e) und/oder herstellungsbedingte Verunreinigungen enthält (Dicksaft),

mit mindestens 0,1 Gew.-% Impfkristallen des zu gewinnenden $C_{5/6}$-Anhydrozuckeralkohols animpft,

einen erheblichen Teil des in der eingesetzten Lösung enthaltenen zu gewinnenden $C_{5/6}$-Anhydrozuckeralkohols bei einer Temperatur von höchstens 70°C auskristallisieren läßt, wobei man die Übersättigung der Lösung (Mutterlauge) in Bezug auf den zu kristallisierenden $C_{5/6}$-Anhydrozuckeralkohol durch

Verdampfen von Wasser, Zufuhr von Dicksaft und/oder langsame Temperatursenkung mit einer Geschwindigkeit von höchstens 0,1°C/Minute

in einem Bereich hält, in dem die vorhandenen Kristalle weiterwachsen, jedoch praktisch keine Spontankristallkeimbildung erfolgt,

und

den auskristallisierten $C_{5/6}$-Anhydrozuckeralkohol von der Mutterlauge abtrennt und

die abgetrennten Kristalle gegebenenfalls mit möglichst wenig Wasser oder einer wäßrigen Lösung des betreffenden $C_{5/6}$-Anhydrozuckeralkohols wäscht und/oder trocknet.

2. Verfahren nach Anspruch 1, dadurch gekennzeichnet, daß die von den $C_{5/6}$-Anhydrozuckeralkoholkristallen abgetrennte Mutterlauge, gegebenenfalls nach Einengung, zumindest teilweise

einer Umsetzung von $C_{5/6}$-Zuckeralkohol(en) in $C_{5/6}$-Anhydrozuckeralkohol(e) und/oder von Mono- in Dianhydrohexit(e) durch säurekatalysierte Wasserabspaltung unterworfen und/oder

als Dicksaft mindestens einer weiteren Kristallisation zu- und/oder zurückgeführt wird, in der der gleiche wie oder ein anderer $C_{5/6}$-Anhydrozuckeralkohol als der bei der ersten bzw. der vorhergegangenen Kristallisation gewonnene auskristallisiert wird.

3. Verfahren zur Gewinnung mindestens zweier kristalliner $C_{5/6}$-Anhydrozuckeralkohole nach Anspruch 2, dadurch gekennzeichnet, daß

von einem Dicksaft ausgegangen wird, der nach der Abtrennung eines ersten kristallinen $C_{5/6}$-Anhydrozuckeralkohols eine Mutterlauge liefert, die, bezogen auf Trockensubstanz, mindestens 40 Gew.-% mindestens eines zweiten $C_{5/6}$-Anhydrozuckeralkohols und/oder durch säurekatalysierte Wasserabspaltung in den zweiten $C_{5/6}$-Anhydrozuckeralkohol überführbare(r) Verbindung(en) enthält, und daß man die gegebenenfalls aufkonzentrierte und/oder einer säurekatalysierten Wasserabspaltung unterworfene Mutterlauge aus der ersten Kristallisation mit mindestens 0,1 Gew.-% kristallinem zweitem $C_{5/6}$-Anhydrozuckeralkohol animpft.

4. Verfahren nach einem der Ansprüche 1 bis 3, dadurch gekennzeichnet, daß ein Dicksaft mit einem Wassergehalt von mindestens 1, vorzugsweise 2 und insbesondere 3 Gewichtsprozent verwendet wird.

5. Verfahren nach Anspruch 4, dadurch gekennzeichnet, daß ein Dicksaft mit einem Wassergehalt von höchstens 20, vorzugsweise höchstens 15 und insbesondere nicht mehr als 8 Gew.-% verwendet wird.

6. Verfahren nach einem der Ansprüche 1 bis 5, dadurch gekennzeichnet, daß ein Dicksaft verwendet wird, dessen Gehalt an zu kristallisierendem $C_{5/6}$-Anhydrozuckeralkohol mindestens 50, vorzugsweise mehr als 60 und insbesondere mindestens 75 Gew.-%, bezogen auf Trockensubstanz, beträgt.

7. Verfahren nach einem der Ansprüche 1 bis 6, dadurch gekennzeichnet, daß ein Dicksaft verwendet wird, der, bezogen auf Trockensubstanz, bis zu 99, vorzugsweise bis zu 95 und insbesondere bis zu 90 Gew.-% des zu kristallisierenden $C_{5/6}$-Anhydrozuckeralkohols enthält.

8. Verfahren nach einem der Ansprüche 1 bis 7, dadurch gekennzeichnet, daß der Dicksaft mit 0,2 bis 20, vorzugsweise 0,5 bis 10 und insbesondere 1 bis 5 Gew.-% Impfkristallen angeimpft wird.

9. Verfahren nach einem der Ansprüche 1 bis 8, dadurch gekennzeichnet, daß man die Kristallisation bei einer Temperatur im Bereich von 20 bis 65, vorzugsweise 25 bis 60 und insbesondere 30 bis 55°C durchführt.

10. Verfahren nach einem der Ansprüche 1 bis 9, dadurch gekennzeichnet, daß man die Kristallisation, zumindest in der Endphase, unter allmählichem Absenken der Temperatur, vorzugsweise mit einer Geschwindigkeit von 0,01 bis 0,05° C/Minute durchführt.

11. Verfahren nach einem der Ansprüche 1 bis 10, dadurch gekennzeichnet, daß man als Dicksaft ein durch säurekatalysierte Wasserabspaltung aus Pentit(en), Hexit(en) und/oder Monoanhydrohexit(en), insbesondere ein unter Verwendung heterogener saurer Katalysatoren hergestelltes, gegebenenfalls in üblicher Weise gereinigtes, entfärbtes und/oder auf einen Wassergehalt von höchstens 30 Gew.-% eingestelltes Reaktionsproduktgemisch einsetzt.

**Claims**

1. A process for the recovery of pure crystalline anhydropentites, mono- and/or dianhydrohexites ($C_{5/6}$-anhydro-sugar alcohols) through crystallization from a solution characterized by inoculating

an aqueous solution with a dry substance content of at least 70 % by weight, which, based on dry substance, contains at least 40 % by weight of the $C_{5/6}$-anhydro-sugar alcohol and optionally as balance one or more other $C_{5/6}$-sugar-alcohol(s), $C_{5/6}$-anhydro-sugar alcohol(s) and/or incidental impurities (thick juice)

with at least 0.1 % by weight of inoculation crystals of the $C_{5/6}$-anhydro-sugar alcohol to be recovered,

allowing a substantial portion of the $C_{5/8}$-anhydro-sugar alcohol to be recovered and contained in the solution used to crystallize out at a temperature of at most 70° C, the over-saturation of the solution (mother liquor) with respect to the $C_{5/6}$-anhydro-sugar alcohol being maintained by

volatilization of water, supply of thick juice and/or a slow decrease in temperature at a rate of at most 0.1° C/minute

within a range, in which the crystals that are present continue to grow, but substantially no spontaneous (crystal) nucleation takes place, and

separating the crystallized $C_{5/6}$-anhydro-sugar alcohol from the mother liquor and washing and/or drying the separated crystals optionally with as little water as possible or an aqueous solution of the particular $C_{5/6}$-anhydro-sugar alcohol.

2. Process according to claim 1, characterized in that the mother liquor separated from the $C_{5/6}$-anhydro-sugar alcohol crystals is, optionally after concentration, subjected at least in part

to a reaction of $C_{5/6}$-sugar alcohol(s) into $C_{5/6}$-anhydro-sugar alcohol(s) and/or of mono- into dianhydrohexite(s) through acid-catalyzed splitting off of water, and/or is fed and/or recycled as thick juice to at least one further crystallization, in which there is crystallized out same or another $C_{5/6}$-anhydro-sugar alcohol from that recovered in the case of the first or, resp., the precedig crystallization.

3. Process for the recovery of at least two crystalline $C_{5/6}$-anhydro-sugar alcohols according to claim 2, characterized in that it is proceeded from a thick juice which, after separation of a first crystalline $C_{5/6}$-anhydro-sugar alcohol, provides a mother liquor which, based upon dry substance, contains at least 40 % by weight of at least a second $C_{5/6}$-anhydro-sugar alcohol and/or (a) compound(s) adapted to be converted through acid-catalyzed splitting off of water into the second $C_{5/6}$-anhydro-sugar alcohol, and in that the mother liquor from the first crystallization is, optionally concentrated and/or subjected to an acid-catalyzed splitting off of water, inoculated with at least 0.1 % by weight of crystalline, second $C_{5/6}$-anhydro-sugar alcohol.

4. Process according to one of claims 1 to 3, characterized by using a thick juice having a water content of at least 1, preferably of 2, and especially of 3 % by weight.

5. Process according to claim 4, characterized by using a thick juice having a water content of at most 20, preferably of 15 and especially of not more than 8 % by weight.

6. Process according to one of claims 1 to 5, characterized by using a thick juice, whose content of $C_{5/6}$-anhydro-sugar alcohol to be crystallized is at least 50, preferably more than 60 and especially at least 75 % by weight, based upon dry substance.

7. Process according to one of claims 1 to 6, characterized by using a thick juice which, based upon dry substance, contains up to 99, preferably up to 95, and especially up to 90% by weight of the $C_{5/6}$-anhydro-sugar alcohol to be crystallized.

8. Process according to one of claims 1 to 7, characterized that the thick juice is inoculated with from 0.2 to 20, preferably from 0.5 to 10, and particularly from 1 to 5 % by weight of inoculation crystals.

9. Process according to one of claims 1 to 8, characterized by carrying crystallization out at a temperature in the range from 20 to 65, preferably from 25 to 60, and especially from 30 to 55° C.

10. Process according to one of claims 1 to 9, characterized by carrying crystallization out, at least in the end phase, under a gradual lowering of the temperature, preferably at a rate of from 0.01 to 0.05° C/minute

11. Process according to one of claims 1 to 10, characterizedby using as the thick juice a reaction product mixture prepared through the acid-catalyzed splitting off of water from pentite(s), hexite(s) and/or monoanhydrohexite(s), in particular one prepared under utilization of heterogeneous acid catalysts, optionally purified in customary manner, de-colored and/or adjusted to a water content of at most 30 % by weight.

**Revendications**

1. Procédé de production d'anhydropentitols, de mono- et/ou de dianhydrohexitols cristallisés purs (anhydrosucre-alcools en $C_{5/6}$) par cristallisation dans une solution, caractérisé en ce que:

on ensemence avec au moins 0,1 % en poids de germes cristallins de l'anhydrosucre-alcool en $C_{5/6}$ que l'on veut obtenir, une solution aqueuse ayant une teneur en matière sèche d'au moins 70 % en poids, qui sur base sèche, contient au moins 40 % en poids de l'anhydrosucre-alcool en $C_{5/6}$ devant cristalliser et comme reste, le cas échéant, un ou plusieurs autres sucres-alcools en $C_{5/6}$, anhydrosucre-alcools en $C_{5/6}$ et/ou impuretés provenant de la préparation (jus dense),

on fait cristalliser une partie importante de l'anhydrosucre-alcool en $C_{5/6}$ désiré, contenu dans la solution utilisée, à une température au maximum égale à 70°C, en maintenant la sur-saturation de la solution (liqueur-mère) par rapport à l'anhydrosucre-alcool en $C_{5/6}$ devant cristalliser par évaporation d'eau, apport de jus dense et/ou lent abaissement de la température à une vitesse au maximum égale à 0,1°C/minute, dans un intervalle dans lequel les cristaux présents continuent de croître, mais pratiquement aucune formation spontanée de germes cristallins ne s'effectue, et

on sépare l'anhydrosucre-alcool en $C_{5/6}$ cristallisé de la liqueur-mère et

on lave les cristaux séparés, le cas échéant, avec, aussi peu d'eau que possible ou avec une solution aqueuse de l'anhydrosucre-alcool en $C_{5/6}$ en question et/ou on sèche.

2. Procédé suivant la revendication 1, caractérisé en ce que la liqueur-mère séparée des cristaux d'anhydrosucre-alcool en $C_{5/6}$ est soumise, éventuellement après concentration, au moins partiellement à une transformation de sucre-alcool(s) en $C_{5/6}$ en anhydrosucre-alcool(s) en $C_{5/6}$ et/ou de mono- en dianhydrohexitol(s) par élimination d'eau catalysée par un acide

et/ou

on l'envoie et/ou on la recycle comme jus dense dans au moins une autre opération de cristallisation dans laquelle cristallise le même anhydrosucre-alcool en $C_{5/6}$ que celui qui a été obtenu lors de la première cristallisation ou de la cristallisation précédente, ou un autre.

3. Procédé de production d'au moins deux anhydrosucre-alcools en $C_{5/6}$ cristallisés suivant la revendication 2, caractérisé en ce qu'on part d'un jus dense qui donne, après séparation d'un premier anhydrosucre-alcool en $C_{5/6}$ cristallisé, une liqueur-mère qui, sur base sèche, contient au moins 40 % en poids d'au moins un second anhydrosucre-alcool en $C_{5/6}$ et/ou d'un ou plusieurs composés pouvant être transformés en le second anhydrosucre-alcool en $C_{5/6}$ par élimination d'eau catalysée par un acide, et en ce qu'on ensemence avec au moins 0,1 % en poids de second anhydrosucre-alcool en $C_{5/6}$ cristallisé, la liqueur-mère éventuellement concentrée et/ou soumise à une élimination d'eau catalysée par un acide, provenant de la première cristallisation.

4. Procédé suivant l'une des revendications 1 à 3, caractérisé en ce qu'on utilise un jus dense ayant une teneur en eau d'au moins 1, de préférence 2 et notamment 3 % en poids.

5. Procédé suivant la revendication 4, caractérisé en ce qu'on utilise un jus dense ayant une teneur en eau au maximum égale à 20, de préférence au maximum égale à 15 et ne dépassant pas notamment 8 % en poids.

6. Procédé suivant l'une des revendications 1 à 5, caractérisé en ce qu'on utilise un jus dense dont la teneur en anhydrosucre-alcool en $C_{5/6}$ devant cristalliser s'élève à au moins 50, de préférence à plus de 60 et notamment à au moins 75 % en poids sur base sèche.

7. Procédé suivant l'une des revendications 1 à 6, caractérisé en ce qu'on utilise un jus dense qui contient, sur base sèche, jusqu'à 99, de préférene jusqu'à 95 et notamment jusqu'à 90 % en poids de l'anhydrosucre-alcool en $C_{5/6}$ devant cristalliser.

8. Procédé suivant l'une des revendications 1 à 7, caractérisé en ce qu'on ensemence le jus dense avec 0,2 à 20, de préférence 0,5 à 10 et notamment à 5 % en poids de germes cristallins.

9. Procédé suivant l'une des revendications 1 à 8, caractérisé en ce qu'on conduit la cristallisation à une température comprise dans l'intervalle de 20 à 65, de préférence 25 à 60 et notamment 30 à 55°C.

10. Procédé suivant l'une des revendications 1 à 9, caractérisé en ce qu'on conduit la cristallisation, tout au moins dans la phase finale, avec abaissement progressif de la température, de préférence à une vitesse de 0,01 à 0,05°C/minute.

11. Procédé suivant l'une des revendications 1 à 10, caractérisé en ce qu'on utilise comme jus dense un mélange de produits réactionnels obtenu par élimination d'eau, catalysée par un acide, de pentitol(s), d'hexitol(s) et/ou de mono-anhydrohexitol(s), notamment en utilisant des catalyseurs acides hétérogènes, éventuellement purifié, décoloré et/ou amené à une teneur en eau de 30 % en poids au maximum, par des moyens classiques.